(19) Europäisches Patentamt
European Patent Office
Office européen des brevets

(11) Numéro de publication : **0 432 068 A1**

(12) **DEMANDE DE BREVET EUROPEEN**

(21) Numéro de dépôt : **90440104.9**

(22) Date de dépôt : **21.11.90**

(51) Int. Cl.$^5$ : **C07C 223/02,** C07C 209/78, C07D 211/80, C07D 265/28, C07D 295/10

(30) Priorité : **07.12.89 FR 8916370**

(43) Date de publication de la demande : **12.06.91 Bulletin 91/24**

(84) Etats contractants désignés : **AT BE CH DE DK ES GB GR IT LI LU NL SE**

(71) Demandeur : **UNIVERSITE DE ROUEN**
**Faculté des Sciences et des Techniques, Laboratoire de Synthèse Organique, BP 118**
**F-76134 Mont-Saint-Aignan Cédex (FR)**

(72) Inventeur : **Le Gailliard, Joel**
**5 rue de Blainville**
**F-76000 Rouen (FR)**
Inventeur : **Combret, Jean-Claude**
**11 rue Louis Dubreuil**
**F-76000 Rouen (FR)**
Inventeur : **Klein, Jean-Louis**
**7 Parc de la Saane**
**F-76130 Mont Saint-Aignan (FR)**

(74) Mandataire : **Arbousse-Bastide, Jean-Claude Philippe**
**CABINET ARBOUSSE BASTIDE 20, rue de Copenhague**
**F-67000 Strasbourg (FR)**

(54) **Procédé d'obtention d'alpha-aminoaldéhydes alpha-éthyléniques et composés obtenus par ce procédé.**

(57) Procédé d'obtention d'α-aminoaldéhydes α-éthyléniques de formule générale :

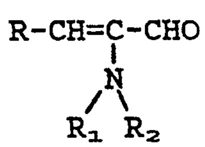

(I)

et nouveaux α-aminoaldéhydes α-éthyléniques obtenus par ce procédé, de formule générale :

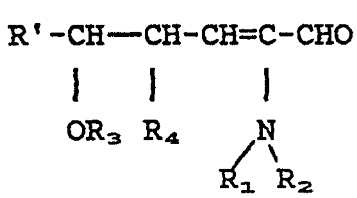

(III)

EP 0 432 068 A1

## PROCEDE D'OBTENTION D'ALPHA-AMINOALDEHYDES ALPHA-ETHYLENIQUES ET NOUVEAUX COMPOSES OBTENUS PAR CE PROCEDE

La présente invention a pour objet un nouveau procédé d'obtention d'α-aminoaldéhydes α-éthyléniques ainsi que de nouveaux composés obtenus par ce procédé.

On sait que les α-aminoaldéhydes α-éthyléniques ne sont pas aisément accessibles par action directe d'amines secondaires sur les aldéhydes α-éthyléniques α-halogénés correspondants, la réaction conduisant à un mélange de composés difficiles à isoler.

On sait également l'intérêt que présentent les α-aminoaldéhydes α-éthyléniques, notamment en ce qu'ils constituent la forme protégée d'α-cétoaldéhydes, lesquels peuvent être des intermédiaires de synthèse très intéressants.

Dans cette perspective, la Demanderesse a récemment mis au point une méthode d'obtention de nouveaux aldéhydes α-éthyléniques α-halogénés qui font l'objet de sa demande de brevet N° 89/14882, et qui peuvent ouvrir une nouvelle voie d'accès à toute une famille de macrolides naturels ou synthétiques, à condition de mettre au point un procédé autorisant la transformation de ces composés en les α-aminoaldéhydes α-éthyléniques correspondants.

Les recherches effectuées dans ce sens par la Demanderesse l'ont conduite à la découverte que la technique du transfert de phase peut être appliquée à la synthèse sélective des α-aminoaldéhydes α-éthyléniques, sous réserve de la mettre en oeuvre dans des conditions bien spécifiques qu'elle a mises au point.

Cette technique pouvant s'appliquer à tous les α-aminoaldéhydes α-halogénés, la présente invention a pour premier objet un procédé d'obtention d'α-aminoaldéhydes α-éthyléniques de formule générale :

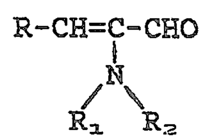

(I)

dans laquelle $R_1$ et $R_2$ désignent chacun un radical alkyle ou, ensemble avec l'atome d'azote, un radical pyrrolidino, pipéridino ou morpholino, et R désigne un radical alkyle ou un radical de formule :

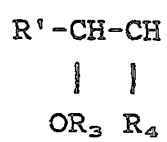

(I')

dans laquelle R' désigne un radical alkyle, aryle ou hétéroaryle, un radical alkyléthylénique ou aryléthylénique ou un radical méthoxy ou éthoxy, $R_3$ désigne un atome d'hydrogène ou un radical méthyle et $R_4$ un atome d'hydrogène ou un radical alkyle linéaire renfermant de 1 à 4 atomes de carbone, ce procédé consistant à faire agir une amine secondaire de formule générale $HNR_1R_2$ sur un aldéhyde α-éthylénique α-halogéné de formule générale :

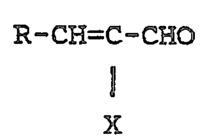

(II)

dans laquelle X désigne un atome d'halogène et R répond à la définition précédemment indiquée, la réaction étant effectuée à température ambiante et dans les conditions usuelles du transfert de phase, c'est-à-dire en milieu biphasé eau-solvant organique, en présence d'une base soluble dans l'eau et d'un agent de transfert jouant le rôle de catalyseur.

La réaction est préférentiellement réalisée en milieu biphasé eau-dichlorométhane, en présence d'acétate de potassium et de bromure de tétrabutylammonium comme catalyseur, les résultats les plus satisfaisants étant obtenus dans ces conditions réactionnelles.

Conformément à l'invention, le milieu biphasé mis en oeuvre est constitué d'un volume d'eau pour environ

trois volumes de dichlorométhane et l'amine secondaire est mise en oeuvre en excès par rapport aux quantités stoechiométriques, et de préférence à raison d'environ 2 équivalents molaires, l'acétate de potassium étant mis en oeuvre à raison de 10 g pour 10 ml d'eau.

La réaction est prolongée pendant un temps assez long, de l'ordre de 3 à 5 heures, sous vive agitation, et l'α-aminoaldéhyde α-éthylénique est isolé du milieu réactionnel par extraction à l'éther éthylique, séchage sur sulfate de magnésium anhydre et distillation.

Le procédé selon l'invention conduit à de bons rendements, de l'ordre de 70% à condition que soit scrupuleusement respecté le protocole réactionnel, lequel consiste en la succession d'opérations suivantes :

- dissolution dans l'eau de l'acétate de potassium ;
- introduction d'une quantité catalytique de bromure de tétrabutylammonium (0,1 à 0,15 équivalent molaire) ;
- addition d'aldéhyde α-éthylénique α-halogéné en solution dans environ les 3/4 du dichlorométhane ;
- agitation énergique du mélange ;
- introduction lente, sous agitation, de l'amine secondaire en solution dans le reste de dichlorométhane ;
- agitation pendant 3 à 5 heures ;
- isolement de l'α-aminoaldéhyde α-éthylénique du milieu réactionnel.

Cette dernière étape du procédé selon l'invention s'effectue de préférence par addition successive, au milieu réactionnel, d'éther éthylique et d'une petite quantité d'eau, suivie de séparation de la phase organique que l'on sèche à l'aide de sulfate de magnésium anhydre, élimination du solvant, après filtration, par distillation sous vide, puis distillation du résidu.

Le procédé selon l'invention constitue un moyen d'accès très avantageux à des intermédiaires clés de synthèse de structure générale :

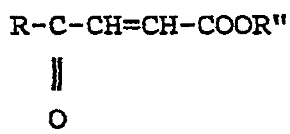

qui, dans le cas où R répond à la formule (I') explicitée ci-dessus, peuvent être utilisés à la synthèse soit de macrolides naturels comme la pyrénophorine, la norpyrénophorine ou la vermiculine, soit de macrolides synthétiques nouveaux.

La présente invention a également pour objet les nouveaux α-aminoaldéhydes α-éthyléniques de formule générale :

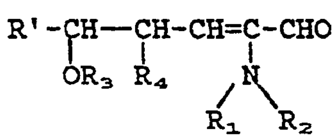

(III)

dans laquelle les différents substituants répondent aux définitions précédemment indiquées, obtenus par le procédé qui constitue le premier objet de l'invention à partir des aldéhydes α-éthyléniques α-halogénés correspondants.

Les composés de formule (III) constituent des intermédiaires de synthèse d'un grand intérêt, ouvrant une nouvelle voie d'accès aux macrolides naturels ainsi qu'à de nouveaux macrolides synthétiques et aux composés polyéthyléniques fonctionnels.

La présente invention se trouve illustrée par les exemples qui suivent et qui en décrivent l'application, étant bien entendu que ces exemples ne présentent aucun caractère limitatif vis-à-vis de l'invention.

## EXEMPLE 1.

Préparation du 2-morpholinobut-2-ènal

Dans un réacteur de 125 ml, on introduit une solution de 10 g d'acétate de potassium anhydre dans 10 $cm^3$ d'eau, puis 0,45 g de bromure de tétrabutylammonium puis une solution de 1,49 g de 2-bromobut-2-ènal dans 25 $cm^3$ de dichlorométhane. On agite violemment et on ajoute lentement une solution de 1,74 g de mor-

pholine dans 5 $cm^3$ de dichlorométhane. On agite durant 4 heures puis on ajoute successivement 60 $cm^3$ d'éther éthylique et 20 $cm_3$ d'eau. On recueille la phase organique que l'on lave avec 3 fois 20 $cm^3$ d'eau ; on ajoute 5 g de sulfate de magnésium, filtre et élimine le solvant par distillation sous vide puis distille le résidu.

On obtient 1,09 g d'un mélange des deux stéréoisomères Z (70%) et E (30%), dont la température d'ébullition est de 58°C sous 0,1 mm de mercure et dont la structure est confirmée par analyse RMN. Le rendement de la réaction est de 70%.

EXEMPLE 2.

Préparation du 5-méthoxy-2-morpholinohex-2-ènal.

On procède de la même manière qu'à l'exemple 1, en mettant en oeuvre 10 g d'acétate de potassium dans 10 $cm^3$ d'eau, 2,07 g de 2-bromo-5-méthoxyhex-2-ènal dans 25 $cm^3$ de dichlorométhane, 0,47 g de bromure de tétrabutylammonium et 1,74 g de morpholine en solution dans 5 $cm^3$ de dichlorométhane.

La réaction, prolongée pendant 5 heures, conduit à l'obtention de 1,52 g de produit brut, de pureté 90%, dont la structure est confirmée par analyse RMN.

Le rendement de la réaction est de 64%.

EXEMPLE 3.

Préparation du 7-méthoxy-4-oxooct-2-ènoate d'éthyle.

Cet exemple illustre l'utilisation du composé de l'exemple 2 pour obtenir un intermédiaire de synthèse connu et particulièrement intéressant car constituant une voie d'accès à la pyrénophorine.

Dans un réacteur de 50 ml, sous atmosphère inerte, on introduit 0,17 g d'hydrure de sodium en solution dans 10 $cm^3$ d'éther éthylique anhydre. On refroidit à 0°C puis ajoute goutte à goutte 1,52 g de phosphonoacétate de triéthyle. On maintient à 0°C durant 30 mn, laisse revenir à température ambiante puis ajoute rapidement 1,52 g de 5-méthoxy-2-morpholinohex-2-ènal en solution dans 5 $cm^3$ d'éther éthylique anhydre. On porte à reflux pendant 45 mn, refroidit à température ambiante, puis ajoute 10 $cm^3$ d'une solution aqueuse molaire d'acide chlorydrique. On recueille la phase organique et extrait 2 fois avec 10 $cm^3$ d'éther éthylique la phase aqueuse. On rassemble les fractions organiques, ajoute 5 g de sulfate de magnésium anhydre, filtre et élimine le solvant sous vide dans un évaporateur rotatif puis on distille le résidu sous pression réduite.

On obtient 0,64 g, ce qui représente un rendement global de 28% à partir du bromo-2-but-2-ènal. La structure du produit obtenu, de température d'ébullition 78°C sous o,1 mm de mercure, est confirmée par analyse RMN et IR.

## Revendications

1. Procédé d'obtention d'α-aminoaldéhydes α-éthyléniques de formule générale :

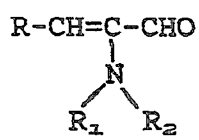

(I)

dans laquelle $R_1$ et $R_2$ désignent chacun un radical alkyle ou, ensemble avec l'atome d'azote, un radical pyrrolidino, pipéridino ou morpholino, et R désigne un radical alkyle ou un radical de formule

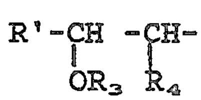

(I')

dans laquelle R' désigne un radical alkyle, aryle ou hétéroaryle, un radical alkyléthylénique ou aryléthylénique ou un radical méthoxy ou éthoxy, $R_3$ désigne un atome d'hydrogène ou un radical méthyle et $R_4$ un atome d'hydrogène ou un radical alkyle linéaire renfermant de 1 à 4 atomes de carbone, caractérisé en ce qu'il consiste à faire agir une amine secondaire de formule générale $HNR_1R_2$ sur un aldéhyle α-éthylénique α-halogéné de formule générale

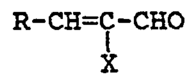

(II)

dans laquelle X désigne un atome d'halogène et R répond à la définition précédemment indiquée, la réaction étant effectuée à température ambiante, en milieu biphasé eau-solvant organique, en présence d'une base soluble dans l'eau et d'un agent de transfert jouant le rôle de catalyseur.

2. Procédé selon la revendication 1, caractérisé en ce que la réaction est réalisée en milieu biphasé eau-dichlorométhane, en présence d'acétate de potassium et de bromure de tétrabutylammonium en quantités catalytiques.

3. Procédé selon la revendication 2, caractérisé en ce que le milieu biphasé est constitué d'un volume d'eau pour environ trois volumes de dichlorométhane.

4. Procédé selon la revendication 2 ou la revendication 3, caractérisé en ce que l'amine secondaire est mise en oeuvre en excès par rapport à la quantité stoechiométrique, à raison d'environ deux équivalents molaires.

5. Procédé selon l'une quelconque des revendications 2 à 4, caractérisé en ce que l'acétate de potassium est mis en oeuvre à raison de 10 grammes pour 10 millilitres d'eau.

6. Procédé selon l'une quelconque des revendications 2 à 5, caractérisé en ce que le bromure de tétrabutylammonium est mis en oeuvre à raison de 0,1 à 0,15 équivalents molaires.

7. Procédé selon l'une quelconque des revendications précédentes, caractérisé en ce que la réaction est prolongée pendant une durée de 3 à 5 heures, sous vive agitation.

8. Procédé selon l'une quelconque des revendications précédentes, caractérisé en ce que l'α-aminoaldéhyde α-éthylénique est isolé du milieu réactionnel par extraction à l'éther éthylique suivie de séchage sur sulfate de magnésium anhydre et distillation.

9. Nouveaux α-aminoaldéhydes α-éthyléniques de formule générale

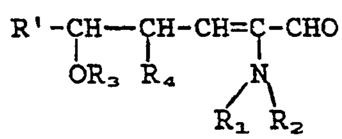

(III)

dans laquelle $R_1$ et $R_2$ désignent chacun un radical alkyle ou, ensemble avec l'atome d'azote, un radical pyrrolidino, pipéridino ou morpholino, R' désigne un radical alkyle, aryle ou hétéroaryle, un radical alkyléthylénique ou aryléthylénique ou un radical méthoxy ou éthoxy, $R_3$ désigne un atome d'hydrogène ou un radical méthyle et $R_4$ un atome d'hydrogène ou un radical alkyle linéaire renfermant de 1 à 4 atomes de carbone.

Office européen des brevets

# RAPPORT DE RECHERCHE EUROPEENNE

Numero de la demande

EP 90 44 0104

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SYNTHESIS JOURNAL OF SYNTHETIC ORGANIC CHEMISTRY no. 6, juin 1989, pages 446,447, Georg Thieme Verlag, Stuttgart, DE; N.A. KEIKO et al.: "A new synthetic route to 2-to 2-dialkylamino-2-alkenals" * pages 446,447 * --- | 1,9 | C 07 C 223/02<br>C 07 C 209/78<br>C 07 D 211/80<br>C 07 D 265/28<br>C 07 D 295/10 |
| A | CHEMICAL ABSTRACTS vol. 109, no. 1, 4 juillet 1988, page 572, abrégé no. 6065r, Columbus, Ohio, US; N.A. KEIKO et al.: "Reaction on alpha-halo-alpha, beta-unsaturated aldehydes with secondary amines. A new method for preparation of N-disubstituted alpha-aminocrotonaldehydes" & Izv. Acad. Nauk SSSR, Ser Khim, 1987, no. 8, pages 1801-1805 --- | 1,9 | |
| A | CHEMICAL ABSTRACTS vol. 105, no. 21, 24 novembre 1986, page 716, abrégé no. 190947w, Columbus, Ohio, US; & SU - 1204619 (IRKUTSK INSTITUTE OF ORGANIC CHEMISTRY) 15.01.1986 --- | 1,3,9 | **DOMAINES TECHNIQUES RECHERCHES (Int. Cl.5)** |
| A | CHEMICAL ABSTRACTS vol. 99, no. 7, 15 août 1983, page 380, abrégé no. 53266s, Columbus, Ohio, US; J.L. KLEIN et al.: "Reaction of secondary amines with alpha,beta-dibromo aldehydes. Preparation of alpha,beta-diamino aldehydes from cinnamaldehyde and new synthesis of 1,2-diaminoehtylenes" & Bull. Soc. Chim. Fr. 1983, no. 1-2, point 2, pages 28-32 --- -/- | 1 | C 07 C 223/00<br>C 07 C 209/00<br>C 07 D 211/00<br>C 07 D 265/00<br>C 07 D 295/00 |

Le présent rapport a été établi pour toutes les revendications

| Lieu de la recherche | Date d'achèvement de la recherche | Examinateur |
|---|---|---|
| BERLIN | 07-01-1991 | RUFET J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 90 44 0104

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS vol. 81, no. 7, 19 août 1974, page 380, abrégé no. 37466s, Columbus, Ohio, US; L. DUHAMEL et al.: "Action of Grignard reagents on formylated enediamines. Access route to alpha-beta-ethylenic alpha-aminoaldehydes" & C.R. Acad. Sci. Ser. C 1974, vo. 278, no. 17, pages 1113-1116 ----- | 1,9 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| BERLIN | 07-01-1991 | RUFET J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)